# EUROPÄISCHE PATENTSCHRIFT

(11) **EP 2 113 237 B1**
(45) Veröffentlichungstag und Bekanntmachung des Hinweises auf die Patenterteilung: **10.12.2014**
(21) Anmeldenummer: 09004859.6
(22) Anmeldetag: 02.04.2009
(51) Int. Cl.: A61K 6/083

(54) **Mit Antiplaque-Wirkstoff(en) ausgestattete Dentalmaterialien**
Dental materials with anti-plaque agents
Matériaux dentaires équipés de matières actives antiplaque

(30) Priorität: 29.04.2008 DE 102008021473
(43) Veröffentlichungstag der Anmeldung: 04.11.2009
(73) Patentinhaber: Heraeus Kulzer GmbH, 63450 Hanau (DE)
(72) Erfinder: Ruppert, Klaus, Dr., 63477 Maintal (DE); Renz, Karl-Heinz, 60489 Frabkfurt (DE); Vogt, Sebastian, Dr., 99092 Erfurt (DE)
(74) Vertreter: Bendele, Tanja

(56) Entgegenhaltungen:
- DE-A1- 10 332 680
- DE-A1- 19 937 093
- DE-A1-102007 040 569
- US-A1- 2005 048 005
- US-A1- 2006 205 838

## Beschreibung

Die Erfindung betrifft mit Antiplaque-Wirkstoff(en) ausgestattete Dentalmaterialien.

### Hintergrund

Polymere Dentalmaterialien, insbesondere auf Acrylat-/Methacrylat-Basis, welche zum dauerhaften Verbleib in die Mundhöhle eingebracht werden, neigen bei mangelnder Oralhygiene zur Plaquebesiedelung an der Werkstoffoberfläche.

Plaque setzt sich aus verschiedenen Bakterien zusammen, die sich durch Proteine und Kohlenhydrate fest auf Oberflächen wie z.B. Zähnen oder dentalen Werkstoffen verankern. Auf dieser ersten Bakterienschicht können sich dann weitere Bakterien festsetzen und damit eine dreidimensionale Kolonie bilden. Durch bestimmte Substanzen, welche von den Bakterien abgegeben werden, ist dieser "Biofilm" nahezu unangreifbar für Antibiotika.

Neben dem hygienischen Aspekt führt Plaque im fortgeschrittenen Stadium auch zu einer starken Verfärbung, so dass es zu ästhetischen Beeinträchtigungen kommt.

### Stand der Technik

Eine Verminderung der Plaque-Besiedlung kann entweder durch Biozide oder durch eine hydrophobe Beschichtung der Dentalwerkstoffe, welche die Anhaftung der Bakterien auf dem Werkstoff verhindert, erfolgen.

Die Verwendung von quaternären Ammoniumsalzen als antimikrobielle Additive ist seit langem bekannt. So wird z.B. Silan mit quaternären Ammoniumgruppen als funktionelle Gruppe von der Firma Microbeshield hergestellt und zur antibakteriellen Ausrüstung von Filtern, Textilien und Wundauflagen vermarktet. Ferner wurden silberhaltige Gläser, Salze oder Zeolithe als antimikrobielle Additive vorgeschlagen.

US 2005/0048005 A1 offenbart antimikrobielle Formulierungen zur Plaque-Reduktion basierend auf kationischen Verbindungen wie u.a. Octenidin. US 2006/0205838 A1 betrifft Dentalmaterial basierend auf Fettsäureestern von Polyhydroxysäuren und einer "Enhancer Component" basierend auf α- oder β-Hydroxysäuren.

### Aufgabenstellung

Ziel der vorliegenden Erfindung ist es, ein Verfahren bereitzustellen, mit dem sich die Besiedelung von Plaque auf dentalen Werkstoffen dauerhaft verhindern bzw. verzögern lässt, ohne die Produkteigenschaften des Dentalmaterials negativ zu beeinflussen.

Hierbei sind folgende Kernanforderungen wichtig:
- Eine homogene Verteilung des Wirkstoffes im Inneren des Materials (Bulk) und auf der Materialoberfläche sollte gewährleistet sein, d.h. umgekehrt: Eine punktuelle Verteilung ist zu vermeiden.
- Der Werkstoff darf nach der Wirkstofffreisetzung keine Mikro- und Makroporen aufweisen (Ästhetik, Rissbildung, Ausgangspunkt und Neubesiedelung).
- Die Inaktivierung des Wirkstoffs auf der Oberfläche soll erschwert sein durch Nachdiffusion aus dem Inneren (Bulk).
- Ein breites Wirkungsspektrum gegen typische Oralkeime ist anzustreben.
- Der Wirkstoff soll retardierend freigesetzt werden.
- Der Wirkstoff soll eine so hohe Freisetzungsrate haben, dass eine antimikrobielle Wirksamkeit gegeben ist, jedoch keine toxischen oder irritierenden/sensibilisierenden Effekte auftreten.
- Der Wirkstoff darf die Polymerisation des Produktes nicht stören oder die Werkstoffeigenschaften negativ beeinflussen, es darf insbesondere keine Phasenseparation (Weichmachereffekt) auftreten.
- Gegenüber dem Wirkstoff sollte nur eine geringwahrscheinliche Resistenzbildung der typischen Oralkeime auftreten.

Octenidin, dessen Herstellung und dessen Antiplaque-Wirkung sind an sich bekannt (Merck Index 14th Ed. 2006, Monograph Number: 0006754). Die Verbindung wird vorwiegend in Mundspülungen eingesetzt. In US 4,206,215 wird auch empfohlen, die dort beschriebenen antimikrobiellen Wirkstoffe in Lacken oder Beschichtungen einzusetzen.

### Erfindung

Erfindungsgemäß wird gemäß Anspruch 1 mit Antiplaque-Wirkstoff ausgestattetes Dentalmaterial vorgeschlagen, das mindestens ein molekulardispers verteiltes Octenidin-Salz oder Dequalinium-Salz enthält, wobei das Salz in gelöster Form in einem oder mehreren der im Dentalbereich üblichen Monomeren auf (Meth-)Acrylat Basis molekulardispers vorliegt, und das Salz mindestens ein Anion aus der Gruppe bestehend aus Fettsäureanionen, Carbonsäure-anionen, Alkyl- und Arylsulfonsäuren, Alkyl- und Arylsulfate aufweist. Weitere bevorzugte Ausgestaltungen sind den abhängigen Ansprüchen zu entnehmen. Die Erfindung betrifft auch ein Verfahren nach Anspruch 15 und eine Verwendung nach Anspruch 14.

Hierbei wird unter Octenidin-Salz eine Verbindung mit der dikationischen Bipyridinium-Grundstruktur nach CAS-Nr. 70775-75-6 verstanden. Darunter sollen auch geringfügige Modifikationen an der chemischen Grundstruktur fallen, z.B. mit Variation der zentralen Alkylkette (n = 7 - 13), Variation der terminalen Alkylketten (n = 4 - 12), und Variation der Anionen. Anstelle des Octenidin-Salzes kann es sich auch um strukturell ähnlich aufgebaute Dequalinium-Salze handeln.

Als Anionen solcher Salze kommen nicht radikalisch polymerisierbare Anionen (z.B. Fettsäureanionen, Carbonsäureanionen, Alkyl- und Arylsulfonsäuren, Alkyl- und Arylsulfate) in Frage. Beispiele dafür sind Hexanoate, Dodecylate, Stearate und Dodecylsulfate.

Solche Octenidinsalze zeigen im Dentalmaterial eine gewisse Mobilität/Migrationsfähigkeit.

Es können auch radikalisch polymerisierbare Anionen ungesättigter Fettsäuren (z.B. Oleate, Sorbate) eingesetzt werden. Diese lassen sich bei der Härtung des Dentalmaterials einpolymerisieren und sind immobil, während der kationische Anteil sich mobilisieren lässt. Es ist auch eine Kombination von Salzen mit Anionen beider Gruppen möglich. Damit ist die Freisetzung via Migration der Salze oder der Kationen aus dem Dentalmaterial mit verschiedenen Geschwindigkeiten möglich. Schließlich ist infolge der Abrasion des Materials selbst eine Restfreisetzung möglich.

Das erfindungsgemäße Dentalmaterial zeichnet sich dadurch aus, dass das Salz im Bereich bis zu einer Obergrenze von 100 °C temperaturstabil ist. Ferner kann das Dentalmaterial ein Salz enthalten, bei dem das Salz mindestens ein nicht radikalisch polymerisierbares Anion aufweist, insbesondere kann das Salz ein löslichkeitsvermittelndes, radikalisch polymerisierbares Anion aufweisen. Auch kann das Dentalmaterial zusätzlich mindestens ein Octenidin- oder Dequalinium-Salz mit einem löslichkeitsvermittelnden, nicht radikalisch polymersierbaren Anion enthalten.

Ebenfalls Gegenstand der Erfindung ist ein Dentalmaterial mit einem Salz, das migrationsfähig im Dentalmaterial in einem Temperaturbereich von 15 - 40 °C ist. Zudem kann das Dentalmaterial eine weitere antimikrobielle Komponente enthalten. Ebenfalls kann das Dentalmaterial eine weitere antimikrobielle Komponente enthalten, wobei die weitere Komponente der Gruppe bestehend aus monokationischen Antiseptika, dikationischen Antiseptika, oligo- oder polymeren kationischen Antiseptika und antiseptischen Schwermetall-Verbindungen entstammt.

Das Octenidin- oder Dequaliniumsalz liegt im Dentalmaterial bevorzugt in Mengen unter 10 Gew.% vor. Das bedeutet insbesondere eine Wirkstofffreisetzung in nicht toxischen, nicht irritierenden und nicht sensibilisierenden, aber antimikrobiell wirksamen Mengen. Außerdem sollten die Mengen so gewählt werden, daß der Wirkstoff in geruchs- und geschmacksneutralen Mengen freigesetzt wird. Verfärbungen des Dentalmaterials sollten ebenfalls durch die Wahl der Menge ausgeschlossen werden. Octenidin- und/oder Dequaliniumsalz sind zB in Mengen von <5, 3 oder <1 Gew% im Dentalmaterial vorhanden.

Zum Einpolymerisieren oder zur homogenen Verteilung im Dentalmaterial wird das Salz zweckmäßig dem entsprechenden Monomer oder der Monomermischung zugesetzt. Beispiele geeigneter Monomere sind die im Dentalbereich üblichen, wie z.B. monomere (Meth)acrylate wie Ethyleneglycoldimethacrylat EDMA, Diethylenglycoldimethacrylat, Triethylenglycoldimethacrylate TEGDMA, Glyceroldimethacrylat GDMA, Glyceroltrimethacrylate, Trimethylolpropantrimethacrylat, Pentaerythritoldimethacrylat, Pentaerythritoltrimeth-acrylat, Pentaerythritoltetramethacrylat, Derivate von Bisphenol A wie Bisphenol-A-dimeth-acrylat und Bisphenol-A-diglycoldimethacrylat, Urethanmethacrylat erhältlich aus Diisocyanaten und Hydroxyalkylmethacrylaten, sowie Reaktionsprodukte von Polyolen, Diisocyanaten und Hydroxyalkylmethacrylaten gemäß DE 37 03 080 A1 oder DE 37 03 120 A1; C₁₋₁₂-, vorzugsweise C₁₋₄-Alkylmethacrylate wie Methylmethacrylat, Ethylmethacrylat, n-Propyl-methacrylat, Isopropylmethacrylat, n-Butylmethacrylat and t-Butylmethacrylat, Hydroxyalkyl-C₁₋₄-methacrylate wie 2-Hydroxyethylmethacrylat, 2-Hydroxypropylmethacrylat, Diethyl-eneglycolmonomethacrylat, Triethylenglycolmonomethacrylat, Alkoxy-C₁₋₄ alkylmethacrylate wie 2-Methoxyethylmethacrylat, 3-Methoxybutylmethacrylat und Ethyltriglycolmethacrylat. Geeignete Monomere sind davon jeweils die Monomere selbst, daraus hergestellte polymerisierbare Präpolymere sowie Mischungen von diesen.

Geeignete Füllstoffe sind insbesondere Quarz- und Glaskeramikpulver, Aluminiumoxide und/oder Siliciumoxide. Dazu gehören sogenannte Mikrofüller, deren Körnung in nm-Bereich liegt, wie etwa hochdisperse feinstteilige pyrogene oder gefällte Kieselsäure und sogenannte Makrofüller, deren Körnung im Mikrometerbereich liegt, insbesondere körnige Kieselsäure oder gemahlene Dentalgläser. Bei den Gläsern handelt es sich etwa um
Bariumglas-, Bariumsilikatglas-, Lithium- oder Aluminium-Silikatglas-Pulver, vorzugsweise um Aluminiumsilikatgläser, die mit Barium, Strontium oder seltenen Erden dotiert sein können (vgl. DE-PS 24 58 380). Bevorzugt sind fein gemahlene Gläser oder Quarz mit mittleren Teilchengrößen zwischen etwa 1 und 10 Mikrometer sowie hochdisperses SiO₂ mit mittleren Teilchengrössen zwischen etwa 10 und 400 nm.

Die Aushärtung der Dentalzusammensetzungen kann je nach Art des verwendeten Polymerisationsinitiators durch thermische, photochemische oder redoxinduzierte radikalische Polymerisation erfolgen.

Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie Azobisisobutyroethylester, Azobisisobutyronitril, Azobis-(2-methylpropionamidin)dihydro-chlorid, Benzpinakol oder 2,2-Dimethylbenzpinakol.

Bevorzugte Photoinitiatoren sind Benzophenon, Benzoin sowie deren Derivate oder alpha- Diketone oder deren Derivate wie 9,10-Phenanthrenchinon, Diacetyl oder 4,4-Dichlorbenzil. Besonders bevorzugt werden Campherchinon und 2,2-Dimethoxy-2-phenylacetophenon und besonders bevorzugt alpha-Diketone in Kombination mit Aminen als Reduktionsmittel, wie z.B. 4-(N,N-Dimethylamino)-benzoesäureester, N,N-Dimethylaminoethylmethacrylat, N,N-Dimethylsym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Als Initiatoren für die bei Raumtemperatur durchgeführte Polymerisation werden vorzugsweise Redox-Initiatorkombinationen, wie z.B. Kombinationen von Benzoyl- oder Lauryl-peroxid mit N,N-Dimethyl-sym.-xylidin oder N,N-Dimethyl-p-toluidin, verwendet.

Die folgenden Beispiele erläutern die Erfindung näher. Teile- und Prozentangaben beziehen sich wie in der übrigen Beschreibung auf das Gewicht, sofern nicht anders angegeben.

### Ausführungsbeispiele

### Herstellen von Octenidin-Palmitat

100 ml Methanol werden in einem Rundkolben vorgelegt, mit einer stöchiometrischen Menge NaOH versetzt und unter Rühren bei RT gelöst. Nach Zugabe der entsprechenden molaren Menge Octenidin-dihydrochlorid wird 10 min gerührt und eine stöchiometrische Menge Palmitinsäure zugegeben. Nach ca. 30 min Rühren wird filtriert und das Lösungsmittel abgezogen

### Herstellen eines Dentalkomposits mit homogen molekulardispers verteiltem Octenidin-Palmitat

Octenidin-Palimitat wird zu einer 70:30 Mischung aus Bis-GMA und TEDMA in einer solchen Menge zugeben, dass die Endkonzentration an Octenidin-Palmitat 3 bzw. 6 Gew. % beträgt. Es wird bis zum vollständigen Lösen des Octenidin-Palmitats unter leichter Erwärmung gerührt.

Zugabe von üblichen Photoinitiatoren und Stabilisatoren sowie 65 Gew. % Dentalglas in einer Korngröße von ∼ 1 µm sowie zur Einstellung der Rheologie ca. 8 Gew. % pyrogene Kieselsäure Aerosil OX50 vervollständigen das Füllungskomposit. Nach Bedarf werden Farbpigmente zur Farbeinstellung zugegeben.

### Härtung zu Prüfkörpern

Das mit dem antimikrobiellen Additiv versehene Komposit Venus@ (Fa. Heraeus Kulzer) wird durch Einstreichen in eine Stahlform zu Prüfplatten von 20 mm Durchmesser und einer Dicke von 1 mm verarbeitet. Bei Belichtung mit der Zahnarztlampe Translux Energy® (Fa. Heraeus Kulzer) entsprechend den Vorgaben aus ISO4049 härtet den Prüfkörper aus.

### Test der antimikrobiellen Wirkung

Folgende sterile Proben wurden in Fließkammern eingesetzt:
Venus® Referenzmaterial ohne Wirkstoff,
Venus® mit 3% Wirkstoff,
Venus® mit 6% Wirkstoff,
   1 x Titan (Kontrolle).

Anschließend wurden die Fließkammern mit einem Bioreaktor gekoppelt, der zur Durchführung der Plaqueadhärenzversuche eine frisch vorbereitete bakterielle Mischkultur (Streptococcus mutans, Streptococcus sanguinis, Actinomyces viscosus, Fusobacterium nucleatum, Veillonella parvula) in der logarithmischen Wachstumsphase mit definierten Anteilen der einzelnen Bakterienspezies enthielt. Die Inkubation der Mischkultur erfolgte entsprechend einer kontinuierlichen Kulturführung anaerob bei 37°C, pH=7,2 und 5,0 g/l Saccharose als C-Quelle. Mittels Schlauchpumpen wurde die bakterielle Mischkultur durch die Fließkammern über die Materialoberfläche geleitet. Die Testung der Biofilmbildung erfolgte über einen Zeitraum von 5 Tagen. Nach Entnahme der Proben aus den Fließkammern erfolgte eine Spülung der Proben zur Entfernung nicht adhärierter Bakterien.

### Analysen:

Die Analyse der Bakterienadhäsion auf den Probenoberflächen erfolgte nach selektiver Fluoreszenzanfärbung adhärierter lebender und toter Mikroorganismen.

### Ergebnisse:

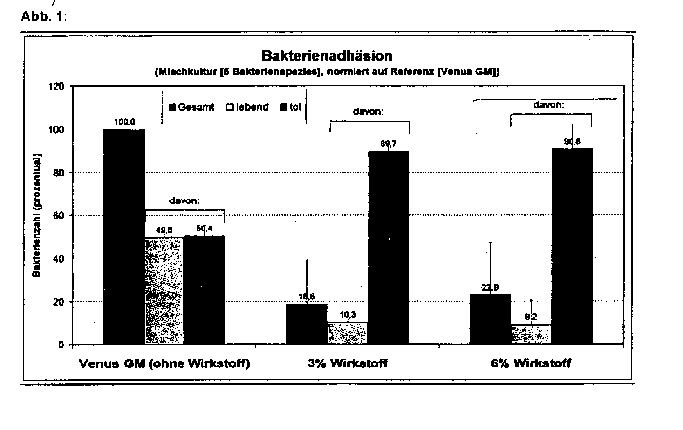

Die Zugabe des Wirkstoffes hat einen deutlichen Effekt auf die Menge adhärierender Bakterien und insbesondere auf die Vitalität der adhärierenden Bakterienpopulation. In Abb. 1 ist die Adhäsion von Plaquebakterien (Mischkultur, 5 Spezies) nach 5 Tagen dynamischer Kultivierung (Fliesskammer) auf Polymermaterialien (Komposit Venus@, Heraeus Kulzer) mit und ohne Wirkstoff dargestellt. Es wurde eine Reduktion der Bakterienzahl um ca. 80% beobachtet.

Das unmodifizierte Polymermaterial weist eine Verteilung lebender und toter Bakterien von ca. 50:50 auf (vgl. Abb. 1). Durch die Wirkstoffzugabe wird das Verhältnis deutlich in Richtung toter Bakterien verschoben (10% lebend, 90% tot). Dies ist ein klarer Beleg für die antibakterielle Wirksamkeit des eingesetzten Additivs.

Die Ergebnisse sind in dem Diagramm der Fig. 1 zusammengestellt.

## Patentansprüche

1. Mit Antiplaque-Wirkstoff ausgestattetes Dentalmaterial, **dadurch gekennzeichnet, dass** es mindestens ein molekulardispers verteiltes Octenidin-Salz oder Dequalinium-Salz enthält, wobei das Salz in gelöster Form in einem oder mehreren der im Dentalbereich üblichen Monomeren auf (Meth-)Acrylat Basis molekulardispers vorliegt und das Salz mindestens ein Anion aus der Gruppe bestehend aus Fettsäureanionen, Carbonsäure-anionen, Alkyl- und Arylsulfonsäuren, Alkyl- und Arylsulfate aufweist.

2. Dentalmaterial nach Anspruch 1, enthaltend eines oder mehrere im Dentalbereich üblichen Monomer(e); mindestens ein molekulardispers gelöstes Octenidin-Salz oder Dequalinium-Salz, sowie mindestens einen Stoff aus der Gruppe der Polymerisationsinitiatoren, Stabilisatoren und Füllstoffen.

3. Dentalmaterial nach Anspruch 1 oder 2, in dem das Salz in einer Konzentration von < 10 Gew. % vorliegt.

4. Dentalmaterial nach einem der vorstehenden Ansprüche, bei dem das Salz im Bereich bis zu einer Obergrenze von 100 °C temperaturstabil ist.

5. Dentalmaterial nach einem der vorstehenden Ansprüche bei dem das Salz mindestens ein nicht radikalisch polymerisierbares Anion aufweist.

6. Dentalmaterial nach einem der vorstehenden Ansprüche, **dadurch gekennzeichnet, dass** das Salz migrationsfähig im Dentalmaterial in einem Temperaturbereich von 15 - 40 °C ist.

7. Dentalmaterial nach einem der vorstehenden Ansprüche, enthaltend das Salz mit einem löslichkeitsvermittelnden, radikalisch polymerisierbaren Anion

8. Dentalmaterial nach Anspruch 7 enthaltend zusätzlich mindestens ein Octenidin-oder Dequalinium-Salz mit einem löslichkeitsvermittelnden, nicht radikalisch polymersierbaren Anion.

9. Dentalmaterial nach Anspruch 7 oder 8, wobei sich die Anionen einpolymerisieren lassen.

10. Dentalmaterial nach einem der vorstehenden Ansprüche, **dadurch gekennzeichnet, dass** die Gesamtmenge an Octenidin-Salz und/oder Dequaliniumsalz < 5 Gew.% beträgt.

11. Dentalmaterial nach Anspruch 10, **dadurch gekennzeichnet, dass** die Menge < 3 Gew.% beträgt.

12. Dentalmaterial nach einem der vorstehenden Ansprüche, das eine weitere antimikrobielle Komponente enthält.

13. Dentalmaterial nach Anspruch 12, wobei die weitere Komponente der Gruppe bestehend aus monokationischen Antiseptika, dikationischen Antiseptika, oligo- oder polymeren kationischen Antiseptika und antiseptischen Schwermetall-Verbindungen entstammt.

14. Verwendung von in einem (Meth-)Acrylat-Monomeren molekulardispers verteilten Octenidin-Salzen oder Dequalinium-Salzen zur Ausstattung von Dentalmaterial mit Antiplaque Eigenschaften wobei das Salz mindestens ein Anion aus der Gruppe bestehend aus Fettsäureanionen, Carbonsäure-anionen, Alkyl- und Arylsulfonsäuren, Alkyl- und Arylsulfate aufweist.

15. Verfahren zur Ausstattung von Dentalmaterial auf (Meth-)Acrylat-Basis mit Antiplaque Eigenschaften, bei dem vor der Polymerisation mindestens ein Octenidin-Salz oder Dequalinium-Salz molekulardispers in einem (Meth-)Acrylat-Monomeren verteilt wird, wobei das Salz mindestens ein Anion aus der Gruppe bestehend aus Fettsäureanionen, Carbonsäure-anionen, Alkyl- und Arylsulfonsäuren, Alkyl- und Arylsulfate aufweist.

## Claims

1. Dental material equipped with anti-plaque agent, **characterised in that** it contains at least one molecular disperse distributed octenidine salt or dequalinium salt, whereby the salt is present dissolved in molecular dispersed form in one or more (meth-)acrylate-based manomer(s) that is/are common in dentistry, and the salt comprises at least one anion from the group consisting of fatty acid anions, carboxylic acid anions, alkyl- and arylsulfonic acids, alkyl- and arylsulfates.

2. Dental material according to claim 1, containing one or more monomer(s) that is/are common in dentistry; at least one octenidine salt or dequalinium salt that is dissolved such as to be molecularly disperse, and at least one substance from the group of polymerisation initiators, stabilisers, and fillers.

3. Dental material according to claim 1 or 2, in which the salt is present at a concentration of < 10 % by weight.

4. Dental material according to any one of the preceding claims, in which the salt is temperature-resistant in the range up to an upper threshold of 100 °C.

5. Dental material according to any one of the preceding claims, in which the salt comprises at least one anion that is not amenable to radical polymerisation.

6. Dental material according to any one of the preceding claims, **characterised in that** the salt is capable of migration in the dental material in a temperature range of 15 - 40 °C.

7. Dental material according to any one of the preceding claims, containing the salt with a solubilising anion that is amenable to radical polymerisation.

8. Dental material according to claim 7, containing, in addition, at least one octenidine salt or dequalinium salt with a solubilising anion that is not amenable to radical polymerisation.

9. Dental material according to claim 7 or 8, whereby the anions can become incorporated by polymerisation.

10. Dental material according to any one of the preceding claims, **characterised in that** the total amount of octenidine salt and/or dequalinium salt is < 5 % by weight.

11. Dental material according to claim 10, **characterised in that** said amount is < 3 % by weight.

12. Dental material according to any one of the preceding claims, containing a further anti-microbial component.

13. Dental material according to claim 12, whereby the further component originates from the group consisting of mono-cationic antiseptics, di-cationic antiseptics, oligomeric or polymeric cationic antiseptics, and antiseptic heavy metal compounds.

14. Use of octenidine salts or dequalinium salts distributed in a (meth-)acrylate monomer such as to be molecularly disperse, for equipping dental materials with anti-plaque properties, whereby the salt comprises at least one anion from the group consisting of fatty acid anions, carboxylic acid anions, alkyl- and arylsulfonic acids, alkyl- and arylsulfates.

15. Method for equipping (meth-)acrylate-based dental materials with anti-plaque properties, in which, before the polymerisation, at least one octenidine salt or dequalinium salt is being distributed in a (meth-)acrylate monomer such as to be molecularly disperse, whereby the salt comprises at least one anion from the group consisting of fatty acid anions, carboxylic acid anions, alkyl- and arylsulfonic acids, alkyl- and arylsulfates.

## Revendications

1. Matériau dentaire équipé d'agent anti-plaque, **caractérisé en ce qu'**il contient au moins un sel d'octénidine ou sel de déqualinium réparti de manière dispersée au niveau moléculaire, dans lequel le sel est présent sous forme dissoute de manière dispersée au niveau moléculaire dans un ou plusieurs des monomères usuels dans le domaine dentaire sur une base de (méth-)acrylate et le sel présente au moins un anion parmi le groupe constitué d'anions d'acide gras, d'anions d'acide carboxylique, d'acides alkyl- et arylsuffoniques, d'alkyl- et aryisulfates.

2. Matériau dentaire selon la revendication 1, contenant un ou plusieurs monomère(s) usuel(s) dans le domaine dentaire ; au moins un sel d'octénidine ou sel de déqualinium dissous de manière dispersée au niveau moléculaire, ainsi qu'au moins une substance parmi le groupe des initiateurs de polymérisation, stabilisateurs et charges.

3. Matériau dentaire selon la revendication 1 ou 2, dans lequel le sel est présent à une concentration de < 10 % en poids.

4. Matériau dentaire selon une des revendications précédentes, dans lequel le sel est stable au niveau de la température dans la plage jusqu'à une limite supérieure de 100°C.

5. Matériau dentaire selon une des revendications précédentes, dans lequel le sel présente au moins un anion non polymérisable par voie radicalaire.

6. Matériau dentaire selon une des revendications précédentes, **caractérisé en ce que** le sel est capable de migration dans le matériau dentaire dans une plage de température de 15 à 40°C.

7. Matériau dentaire selon une des revendications précédentes, contenant le sel avec un anion promoteur de solubilité, polymérisable par voie radicalaire.

8. Matériau dentaire selon la revendication 7, contenant en outre au moins un sel d'octénidine ou sel de déqualinium avec un anion promoteur de solubilité, non polymérisable par voie radicalaire.

9. Matériau dentaire selon la revendication 7 ou 8, dans lequel les anions peuvent être incorporés dans un polymère.

10. Matériau dentaire selon une des revendications précédentes, **caractérisé en ce que** la quantité totale en sel d'octénidine et/ou sel de déqualinium est < 5 % en poids.

11. Matériau dentaire selon la revendication 10, **caractérisé en ce que** la quantité est < 3 % en poids.

12. Matériau dentaire selon une des revendications précédentes, qui contient un autre composant antimicrobien.

13. Matériau dentaire selon la revendication 12, dans lequel l'autre composant provient du groupe constitué d'antiseptiques monocationiques, d'antiseptiques dicationiques, d'antiseptiques cationiques oligo- ou polymères et de composés de métaux lourds antiseptiques.

14. Utilisation de sels d'octénidine ou sels de déqualinium répartis de manière dispersée au niveau moléculaire dans un monomère de (méth-)acrylate pour l'équipement de matériau dentaire avec des propriétés anti-plaque, dans laquelle le sel présente au moins un anion parmi le groupe constitué d'anions d'acide gras, d'anions d'acide carboxylique, d'acides alkyl- et arylsulfoniques, d'alkyl- et arylsulfates.

15. Procédé d'équipement de matériau dentaire à base de (méth-)acrylate avec des propriétés anti-plaque, dans lequel au moins un sel d'octénidine ou sel de déqualinium est réparti de manière dispersée au niveau moléculaire dans un monomère de (méth-)acrylate avant la polymérisation, dans lequel le sel présente au moins un anion parmi le groupe constitué d'anions d'acide gras, d'anions d'acide carboxylique, d'acides alkyl- et arylsulfoniques, d'alkyl- et arylsulfates.
